# EUROPEAN PATENT SPECIFICATION

(11) **EP 2 585 515 B1**
(45) Date of publication and mention of the grant of the patent: **28.11.2018**
(21) Application number: 11729835.6
(22) Date of filing: 21.06.2011
(51) Int. Cl.: C08G 75/02, C08G 75/12, C09J 181/02, C08L 81/02

(54) **POLYTHIOETHER POLYMERS, METHODS FOR PREPARATION THEREOF, AND COMPOSITIONS COMPRISING THEM**
POLYTHIOETHERPOLYMERE, VERFAHREN ZU IHRER HERSTELLUNG UND ZUSAMMENSETZUNGEN DAMIT
POLYMÈRES DE POLYTHIOÉTHER, PROCÉDÉS DE PRÉPARATION DE CEUX-CI ET COMPOSITIONS COMPRENANT CES POLYMÈRES

(30) Priority: 25.06.2010 US 823206
(43) Date of publication of application: 01.05.2013
(73) Proprietor: PRC-Desoto International, Inc., Sylmar, California 91209 (US)
(72) Inventor: KANIA, Charles M., Natrona Heights, Pennsylvania 15065 (US); LIN, Renhe, Lancaster, California 93536 (US); RAO, Chandra B., Valencia, California 91355 (US)
(74) Representative: f & e patent
(86) International application number: PCT/US2011/041214
(87) International publication number: WO 2011/163202

(56) References cited:
- US-A- 4 080 484
- US-A- 4 591 522
- DATABASE CAPLUS [Online] CHEMICAL ABSTRACTS SERVICE, COLUMBUS, OHIO, US; 6 February 2004 (2004-02-06), Okazaki, E.: "Radiation-curable adhesive compositions with good adhesion, curability and storage stability, and adhesive sheets having them.", XP002662423, retrieved from STN Database accession no. 2004:97666 & JP 2004 035734 A (TOAGOSEI CO LTD) 5 February 2004 (2004-02-05)

## Description

### FIELD OF THE INVENTION

The present invention is directed to polythioethers, methods for preparing such polythioethers, and compositions, such as sealant compositions, that include such polythioethers.

### BACKGROUND OF THE INVENTION

Thiol-terminated sulfur-containing compounds are known to be well-suited for use in various applications, such as aerospace sealant compositions, due, in large part, to their fuel-resistant nature upon cross-linking. Other desirable properties for aerospace sealant compositions include low temperature flexibility, short curing time (the time required to reach a predetermined strength) and elevated-temperature resistance, among others. Sealant compositions exhibiting at least some of these characteristics and containing thiol-terminated sulfur-containing compounds are described in, for example, United States Patent Nos. 2,466,963, 4,366,307, 4,609,762, 5,225,472, 5,912,319, 5,959,071, 6,172,179, 6,232,401, 6,372,849 and 6,509,418.

JP 2004035734 discloses a radiation curable pressure sensitive adhesion composition comprising a polythiol compound, a polyene compound and a posphorous compound and/or an N-nitroso compound.

US 4,591,522 relates to a phothosensitive composition comprising the reaction product of an aliphatic unsaturation containing chlorendate and a non-ester containing polythiol, a liquid acrylate monomer or oligomer and at least one phothoinitiator.

US 4,080,484 describes polyenes containing at least two ethylenically unsaturated bonds per molecule formed by reacting in substantially stoichiometric amounts of a primary amine, a a member of the group consisting of a benzenoid-containing dianhydride, acid anhydride and anhydride acid halide and an ethylenically unsaturated alcohol.

Polythioethers that are liquid at room temperature and pressure and that have excellent low temperature flexibility and fuel resistance, such as are disclosed in U.S. Patent No. 6,172,179 ("the '179 patent"), are often desired in aerospace sealant applications, for example. In some cases, such as is described in the '179 patent, it is desirable to utilize a polyfunctionalizing agent, such as a compound having more than two thiols groups, to produce polythioethers having an average functionality of greater than 2. This desire often stems from the tendency of difunctional, *i.e*., linear, polythioethers, to swell upon prolonged exposure to hydrocarbon fuel and other lubricants. A drawback to the use of such polyfunctionalizing agents, however, has been their tendency to compromise other important sealant properties, such as adhesion and elongation. As a result, it would be desirable to provide a polyfunctional polythioether having an average functionality of greater than 2 that has substantially improved sealant properties, such as improved elongation and/or tensile strength (without compromising adhesion), as compared to prior art polyfunctional polythioethers having a functionality greater than 2. The present invention was made in view of the foregoing desire.

### SUMMARY OF THE INVENTION

he present invention is directed to
a polythioether comprising the reaction product of reactants comprising:
a) an isocyanurate-containing trithiol;
b) a polythiol different from (a); and
c) a diene,
wherein the polythiol (b) comprises a dithiol comprising a compound represented by formula:

HS-R-SH

where R is -[(-CH₂-)ₚ-O-]_{q}-(-CH2-)ᵣ- or -[(-CH₂-)ₚ-S-]_{q}-(-CH₂-)ᵣ-, p is an integer having a value ranging from 2 to 6, q is an integer having a value ranging from 1 to 5, and r is an integer having a value ranging from 2 to 10.
polythioethers. These poilythioethers comprise the reaction product of reactants comprising: a) an isocyanurate-containing trithiol; b) a polythiol different from (a); and c) a diene.

In other respects, the present invention is directed to polythioethers represented by the formula (I): 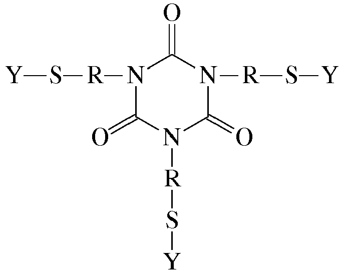 wherein: (A) each Y, which may be the same or different, is a repeat unit comprising a thioether linkage; and (B) each R represents a divalent linking group and may be the same or different, wherein each Y includes the structure:

-[-(CH₂)₂-O-(R²-O)ₘ-(CH₂)₂-S-R¹-S-]ₙ-

wherein:
R¹ is selected from -[(-CH₂)ₚ-X]_{q}-(-CH₂)ᵣ- groups;
R² is selected from C₂ to C₁₀ n-alkylene groups, C₂ to C₆ branched alkylene groups, C₆ to C₈ cycloalkylene groups, C₆ to C₁₄ alkylcycloalkylene groups, heterocyclic groups, and -[(-CH₂)ₚ-X]_{q}-(-CH₂)ᵣ-;
X is selected from O atoms, and S atoms;
p is an integer from 2 to 6;
q is an integer from 1 to 5;
r is an integer from 2 to 10;
m has a value of from 0 to 10; and
n is an integer from 1 to 60.

The present invention is also directed to, *inter alia*, methods for making such polythioethers and compositions, such as sealant compositions, including aerospace sealant compositions, comprising such polythioethers.

### DETAILED DESCRIPTION OF THE INVENTION

For purposes of the following detailed description, it is to be understood that the invention may assume various alternative variations and step sequences, except where expressly specified to the contrary. Moreover, other than in any operating examples, or where otherwise indicated, all numbers expressing, for example, quantities of ingredients used in the specification and claims are to be understood as being modified in all instances by the term "about". Accordingly, unless indicated to the contrary, the numerical parameters set forth in the following specification and attached claims are approximations that may vary depending upon the desired properties to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard variation found in their respective testing measurements.

Also, it should be understood that any numerical range recited herein is intended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10.

As indicated, certain embodiments of the present invention are directed to polythioethers. As used herein, the term "polythioether" refers to compounds comprising at least two thioether linkages, that is "-C-S-C-" linkages. In certain embodiments, such compounds are a polymer. As used herein, "polymer" refers to oligomers and both homopolymers and copolymers. Unless stated otherwise, if used herein, molecular weights are number average molecular weights for polymeric materials indicated as "Mn" and obtained by gel permeation chromatography using a polystyrene standard in an art-recognized manner.

The polythioethers of the present invention comprise the reaction product of reactants comprising: a) an isocyanurate-containing trithiol; b) a polythiol different from (a); and c) a diene.

As used herein, the term "isocyanurate-containing trithiol" refers to a compound that has the structure (II): 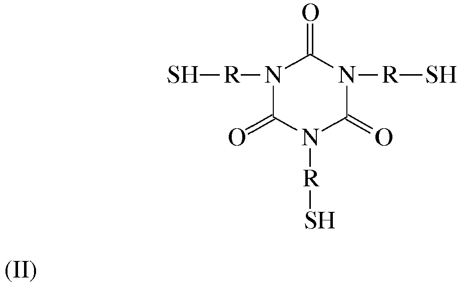 wherein each R represents a divalent linking group and may be the same or different. As used herein, "divalent" refers to a substituent group that, as a substituent group, forms two single, covalent bonds. In certain embodiments, the divalent linking group comprises carbon in the linking group backbone, such as is the case with hydrocarbon linking groups. As used herein, the term "hydrocarbon group" encompasses various groups, such as, for example, branched or unbranched, acyclic or cyclic, saturated or unsaturated groups, and can contain from, for example, 2 to 24 (or in the case of an aromatic/heteroaromatic group from 3 to 24), such as 2 to 6 carbon atoms. Non-limiting examples of suitable divalent hydrocarbon linking groups include straight or branched chain alkylenes, such as ethylene, 1,3-propylene, 1,2-propylene, 1,4-butylene, 1,2-butylene, pentylene, hexylene, heptylene, octylene, nonylene, decylene, undecylene, octadecylene and icosylene. Non-limiting examples of suitable divalent hydrocarbon linking groups also include cyclic alkylenes, such as cyclopentylene, cyclohexylene, cycloheptylene, cyclooctylene, and alkyl-substituted derivatives thereof.

Specific, but non-limiting, examples of compounds having the structure (II), and methods for their preparation, include those compounds described in United States Patent No. 3,676,440 at col. 1, line 50 to col. 5, line 60, United States Patent No. 4,266,055 at col. 7, lines 13 to 30, and United States Patent No. 4,791,185 at col. 1, line 67 to col. 2, line 15 and col. 2, lines 37 to 47. In certain embodiments, however, it is desirable that the isocyanurate-containing trithiol be substantially free, or completely free, from ester linkages. As used herein, the term "substantially free" means that the isocyanurate-containing trithiol includes ester linkages, if at all, as an incidental impurity. Any incidental ester linkages is present in an incidental amounts such that they do not affect the properties of the polythioether polymer of the present invention.

In certain embodiments, the divalent linking group comprises a urethane linkage, such as would be the case with the reaction product of an isocyanurate-containing triisocyanate with sufficient amount of a mercapto-functional monohydric alcohol. Examplary suitable isocyanurate-containing triisocyanates include, without limitation, isocyanurates of diisocyanates, such as the isocyanurate of hexamethylene diisocyanate and the isocyanurate of isophorone diisocyanate, including mixtures thereof. Examplary suitable mercapto-functional monohydric alcohols are 2-mercaptoethanol, 1-mercapto-3-propanol, 3-mercapto-2-butanol, and the like, including mixtures thereof.

Two or more different isocyanurate-containing trithiols can be employed if desired.

As indicated earlier, the reactants used to prepare the polymers of the present invention also comprise a polythiol different from the aforedescribed isocyanurate-containing trithiol. Such a polythiol comprises a compound represented by Formula (III):

HS-R-SH (III)

where R in formula (III) denotes a divalent linking group, selected from (v) -[(-CH₂)ₚ-O-]_{q}-(-CH₂-)ᵣ-;₋ or (vi) -[(-CH₂-)ₚ-S-]_{q}-(-CH₂-)ᵣ-, wherein p is an integer having a value ranging from 2 to 6, q is an integer having a value ranging from 1 to 5, r is an integer having a value ranging from 2 to 10.

Examples of specific dithiols suitable for use in preparing the polymers of the present invention include, without limitation, dimercaptodiethylsulfide, dimercaptodioxaoctane, 1,5-dimercapto-3-oxapentane and mixtures thereof. The polythiol material can have one or more pendant groups selected from lower alkyl groups, lower alkoxy groups and hydroxyl groups. Suitable alkyl pendant groups include C₁ -C₆ linear alkyl, C₃ -C₆ branched alkyl, cyclopentyl, and cyclohexyl.

Other examples of specific dithiols suitable for use in preparing polymers of the present invention include dimercaptodiethylsulfide (DMDS) (in formula (III), R is -[(-CH₂-)ₚ-X-]_{q}-(-CH₂-)ᵣ-, wherein p is 2, r is 2, q is 1, X is S); dimercaptodioxaoctane (DMDO) (in formula (III), R is -[(-CH₂-)ₚ-X-]_{q}-(-CH₂-)ᵣ-, wherein p is 2, q is 2, r is 2, X is O); and 1,5-dimercapto-3-oxapentane (in formula (III), R is -[(-CH₂-)ₚ-X-]_{q}-(-CH₂-)ᵣ-, wherein p is 2, r is 2, q is 1, X is O).Two or more different dithiols can be employed if desired.

As indicated earlier, the reactants used to prepare the polymers of the present invention also comprise a diene. As used herein, the term "diene" refers to a compound that has two carbon-carbon double bonds. Non-limiting exemplary dienes include pentadiene, hexadiene, heptadiene, octadiene, nonadiene, decadiene, undecadiene, dodecadiene, tridecadiene, tetradecadiene, pentadecadiene, hexadecadiene, heptadecadiene, octadecadiene, nonadecadiene, icosadiene, heneicosadiene, docosadiene, tricosadiene, tetracosadiene, pentacosadiene, hexacosadiene, heptacosadiene, octacosadiene, nonacosadiene, triacontadiene, 1,6-heptadiene, 1,7-octadiene, 1,8-nonadiene, 1,9-decadiene, 1,10- undecadiene, 1,11-dodecadiene, 1,12-tridecadiene, 1,13-tetradecadiene, and low molecular weight polybutadienes (M_{(w)} less than 1000 g/mol). Non-limiting exemplary cyclic dienes include cyclopentadiene, vinylnorbornene, norbornadiene, ethylidene norbornene, divinylbenzene, dicyclopentadiene or higher ring containing diolefins with or without substituents at various ring positions.

In certain embodiments, however, the diene comprises a compound represented by Formula (IV):

CH₂=CH-O-(-R⁵-O-)ₘ-CH=CH₂ (IV)

where R⁵ in formula (IV) is a C₂₋₆ n-alkylene group, a C₂₋₆ branched alkylene group, a C₆₋₈ cycloalkylene group, a C₆₋₁₀ alkylcycloalkylene group, or -[(-CH₂-)ₚ-O-]_{q}-(-CH₂)ᵣ-, where p is an integer having a value ranging from 2 to 6, q is an integer having a value ranging from 1 to 5, and r is an integer having a value ranging from 2 to 10.

The materials of formula (IV) are divinyl ethers. Suitable divinyl ethers include those compounds having at least one oxyalkylene group, such as from 1 to 4 oxyalkylene groups, *i.e*., those compounds in which m in formula (IV) is an integer from 1 to 4. In some cases, m in formula (IV) is an integer from 2 to 4. It is also possible to employ commercially available divinyl ether mixtures to produce the polymers of the present invention. Such mixtures are characterized by a non-integral average value for the number of oxyalkylene units per molecule. Thus, m in formula (IV) can also take on rational number values between 0 and 10.0, such as between 1.0 and 10.0, between 1.0 and 4.0, or between 2.0 and 4.0.

Suitable polyvinyl ether monomers from which polythioethers of the present invention may be prepared include divinyl ether monomers, such as divinyl ether, ethylene glycol divinyl ether (EG-DVE) (R in formula (IV) is ethylene and m is 1), butanediol divinyl ether (BD-DVE) (R in formula (IV) is butylene and m is 1), hexanediol divinyl ether (HD-DVE) (R in formula (IV) is hexylene and m is 1), diethylene glycol divinyl ether (DEG-DVE) (R in formula (IV) is ethylene and m is 2), triethylene glycol divinyl ether (R in formula (IV) is ethylene and m is 3), tetraethylene glycol divinyl ether (R in formula (IV) is ethylene and m is 4), cyclohexanedimethanol divinyl ether, polytetrahydrofuryl divinyl ether; trivinyl ether monomers, such as trimethylolpropane trivinyl ether; tetrafunctional ether monomers, such as pentaerythritol tetravinyl ether; and mixtures of two or more such polyvinyl ether monomers. The polyvinyl ether material can have one or more pendant groups selected from alkyl groups, hydroxyl groups, alkoxy groups and amine groups.

Useful divinyl ethers in which R in formula (IV) is C₂₋₆ branched alkylene can be prepared by reacting a polyhydroxy compound with acetylene. Exemplary compounds of this type include compounds in which R in formula (IV) is an alkyl-substituted methylene group such as -CH(CH₃)- (for example "PLURIOL®" blends such as PLURIOL®E-200 divinyl ether (BASF Corp. of Parsippany, N.J.), for which R in formula (IV) is ethylene and m is 3.8) or an alkyl-substituted ethylene (for example -CH₂CH(CH₃)- such as "DPE" polymeric blends including DPE-2 and DPE-3 (International Specialty Products of Wayne, N.J.)).

Other useful divinyl ethers include compounds in which R in formula (IV) is polytetrahydrofuryl (poly-THF) or polyoxyalkylene, such as those having an average of about 3 monomer units.

Two or more polyvinyl ether monomers of the formula (IV) can be used if desired.

The polythioethers of the present invention can be prepared by a number of methods, incuding those described in the Examples herein. In certain embodiments, the polythiol and diene materials, and their respective amounts, used to prepare the polythioethers of the present invention are chosen to yield terminal thiol groups. Thus, in some cases, (n or >n, such as n+1) moles of a polythiol, such as a dithiol having the formula (III) or a mixture of at least two different compounds having the formula (III) and about 0.05 to 1 moles, such as 0.1 to 0.8 moles, of an isocyanurate-containing trithiol, are reacted with (n) moles of a diene, such as a divinyl ether having the formula (IV) or a mixture of at least two different compounds having the formula (IV). In certain embodiments, the isocyanurate-containing trithiol is present in the reaction mixture in an amount sufficient to provide a polythioether having an average functionality of from 2.05 to 3, such as 2.1 to 2.8.

The reaction used to make the polythioethers of the present invention may be catalyzed by a free radical catalyst. Suitable free radical catalysts include azo compounds, for example azobisnitrile compounds such as azo(bis)isobutyronitrile (AIBN); organic peroxides, such as benzoyl peroxide and t-butyl peroxide; and inorganic peroxides, such as hydrogen peroxide. The reaction can also be effected by irradiation with ultraviolet light either with or without a radical initiator/photosensitizer. Ionic catalysis methods, using either inorganic or organic bases, *e.g*., triethylamine, may also be used.

As will be appreciated from the foregoing description, the present invention is also directed to polythioethers of the general formula (I), described earlier. In certain embodiments, with reference to general formula (I), each R, which may be the same or different, is a branched or unbranched, acyclic or cyclic, saturated or unsaturated divalent hydrocarbon group that contains from, for example, 2 to 24 (or in the case of an aromatic/heteroaromatic group from 3 to 24), such as 2 to 6 carbon atoms. Non-limiting examples of suitable divalent hydrocarbon linking groups include straight or branched chain alkylenes, such as ethylene, 1,3-propylene, 1,2-propylene, 1,4-butylene, 1,2-butylene, pentylene, hexylene, heptylene, octylene, nonylene, decylene, undecylene, octadecylene and icosylene. Non-limiting examples of suitable divalent hydrocarbon linking groups also include cyclic alkylenes, such as cyclopentylene, cyclohexylene, cycloheptylene, cyclooctylene, and alkyl-substituted derivatives thereof.

Each Y in general formula (I), which may be the same or different, includes the structure (V):

-[-(CH₂)₂-O-(R²-O)ₘ-(CH₂)₂-S-R¹-S-]ₙ- (V)

wherein R¹ is selected from -[(-CH₂)ₚ-X]_{q}-(-CH₂)ᵣ- groups, and R² is selected from C₂ to C₁₀ n-alkylene groups, C₂ to C₆ branched alkylene groups, C₆ to C₈ cycloalkylene groups, C₆ to C₁₄ alkylcyloalkylene groups, heterocyclic groups, and -[(-CH₂)ₚ-X]_{q}-(-CH₂)ᵣ-. In the foregoing, X may be selected from O atoms, S atoms, and -NR₃- groups, where R₃ may be selected from H atoms and methyl groups, p is an integer from 2 to 6, q is an integer from 1 to 5, and r is an integer from 2 to 10. In addition, m in formula (V) has a value of from 0 to 10 and n is an integer from 1 to 60. For example, in some embodiments of the present invention, m is 1, R¹ is n-butylene, and R² is ethylene. In some embodiments of the present invention, Y is: -[-C₂H₄-O-(-C₂H₄-O)₂-C₂H₄-S-(C₂H₄)-O-(C₂H₄)-O-(C₂H₄)-S-]ₙ-H, where n is an integer having a value of from 1-60.

In certain embodiments, the polythioethers of the present invention are uncapped, *i.e*., thiol terminated. That is, each Y in general formula (I), has the structure (VI):

-[-(CH₂)₂-O-(R₂-O)ₘ-(CH₂)₂-S-R₁-S-]ₙ-H (VI)

In certain embodiments, the uncapped polythioether described above is a liquid at room temperature. Moreover, in certain embodiments, the previously described polythioether has a viscosity, at 100% solids, of no more than 500 poise, such as 10-300 or, in some cases, 100-200 poise, at a temperature of about 25°C and a pressure of about 760 mm Hg determined according to ASTM D-2849 §79-90 using a Brookfield CAP 2000 viscometer, as described in the Examples. Any endpoint within the foregoing ranges can also be used.

In certain embodiments, the uncapped polythioether described above has a number average molecular weight of 300 to 10,000 grams per mole, such as 1,000 to 8,000 grams per mole, the molecular weight being determined by gel permeation chromatography using a polystyrene standard. Any endpoints within the foregoing ranges can also be used.

In certain embodiments, the T_{g} of the polythioether of the present invention is not higher than -55°C, such as not higher than -60°C.

In certain embodiments, the polythioethers of the present invention are "capped", *i.e*., they have terminal groups other than unreacted -SH groups, such as - OH, alkyl, such as a C₁₋₁₀ n-alkyl group, alkylene, such as a C₁₋₁₀ n-alkylene group, - NCO, 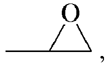 an amine group, or a hydrolyzable functional group, such as a silane group, *e.g*., 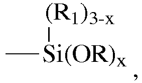 wherein R and R₁ each independently represent an organic group and x is 1, 2, or 3. As indicated, suitable terminal groups include, for example: (i) -OH, such as could be obtained by, for example, (a) reacting an uncapped polythioether of the present invention with a monoxide, such as ethylene oxide, propylene oxide, and the like, in the presence of a base, or (b) reacting an uncapped polythioether of the present invention with an olefinic alcohol, such as, for example, allyl alcohol, or a monovinylether of a diol, such as, for example, ethylene glycol monovinyl ether, propylene glycol monovinyl ether, and the like, in the presence of a free radical initiator; (ii) alkyl, such as could be obtained by reacting an uncapped polythioether of the present invention with an alkylene; (iii) alkylene, such as could be obtained by reacting an uncapped polythioether of the present invention with a diolefin; (iv) -NCO, such as could be obtained by reacting an uncapped polythioether of the present invention with a polyisocyanate; (v) 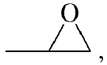 such as could be obtained by reacting an uncapped polythioether of the present invention with a glycidylolefin, wherein the olefinic group may, for example, be an alkylene group or an oxyalkylene group having from 3 to 20, such as 3 to 5, carbon atoms, specific examples of which include allyl glycidyl ether, 1,2-epoxy-5-hexene, 1,2-epoxy-7-octene, 1,2-epoxy-9-decene, 4-vinyl-1-cyclohexene 1,2-epoxide, butadiene monoepoxide, isoprene monoepoxide, and limonene monoepoxide; or (vi) a hydrolyzable functional group, such as could be obtained by reacting an uncapped polythioether of the present invention with an olefinic alkoxysilane, such as vinyltrimethoxysilane, vinyltriethoxysilane, and vinylmethyldimethoxysilane, among others.

As indicated, certain embodiments of the present invention are directed to compositions, such as sealant, coating, and/or electrical potting compositions that include one or more of the previously described polythioethers. As used herein, the term "sealant composition" refers to a composition that is capable of producing a film that has the ability to resist atmospheric conditions, such as moisture and temperature and at least partially block the transmission of materials, such as water, fuel, and other liquid and gasses. In certain embodiments, the sealant compositions of the present invention are useful, *e.g*., as aerospace sealants and linings for fuel tanks. In certain embodiments, the composition comprises a polythioether as described above, a curing agent and a filler.

In certain embodiments, the compositions of the present invention comprise, in addition to a polythioether as described earlier, one or more additional sulfur-containing polymers. As used herein, the term "sulfur-containing polymer" refers to any polymer having at least one sulfur atom, including, but not limited to, polymeric thiols, polythiols, thioethers, polythioethers and polysulfides. A "thiol", as used herein, refers to a compound comprising a thiol or mercaptan group, that is, an "SH" group, either as the sole functional group or in combination with other functional groups, such as hydroxyl groups, as is the case with, for example, thioglycerols. A "polythiol" refers to such a compound having more than one SH group, such as a dithiol or higher functionality thiol. Such groups are typically terminal and/or pendent such that they have an active hydrogen that is reactive with other functional groups. As used herein, the term "polysulfide" refers to any compound that comprises a sulfur-sulfur linkage (-S-S-). A "polythiol" can comprise both a terminal and/or pendant sulfur (-SH) and a non-reactive sulfur atom (-S- or (-S-S-)). Thus, the term "polythiol" generally encompasses "polythioether" and "polysulfide" as well. Suitable sulfur-containing polymers include, for example, those disclosed in U.S. patent numbers 6,172,179, 6,509,418 and 7,009,032. In certain embodiments, therefore, the compositions of the present invention comprise a polythioether that includes a structure having the formula (VII):

-R¹-[-S-(CH₂)₂-O-[-R²-O-]*ₘ*-(CH₂)₂-S-R¹]*ₙ*- (VII)

wherein: (1) R¹ denotes a C₂₋₆ n-alkylene, C₃₋₆ branched alkylene, C₆₋₈ cycloalkylene or C₆₋₁₀ alkylcycloalkylene group, -[(-CH₂-)ₚ-X-]_{q}-(-CH₂-)ᵣ-, or -[(-CH₂-)ₚ-X-]_{q}-(-CH₂-)ᵣ- in which at least one -CH₂- unit is substituted with a methyl group; (2) R² denotes a C₂₋₆ n-alkylene, C₂₋₆ branched alkylene, C₆₋₈ cycloalkylene or C₆₋₁₀ alkylcycloalkylene group, or -[(-CH₂-)ₚ-X-]_{q}-(-CH₂-)ᵣ-, X denotes one selected from the group consisting of O, S and -NR⁶-, R⁶ denotes H or methyl; (3) m is a rational number from 0 to 10; (4) n is an integer from 1 to 60; (5) p is an integer from 2 to 6; (6) q is an integer from 1 to 5, and (7) r is an integer from 2 to 10. Such polythioethers are described in U.S. Patent No. 6,172,179 at col. 2, line 29 to col. 4, line 34.

Any sulfur-containing polymer used according to the present invention can further comprise additional functionality, including but not limited to hydroxyl functionality and epoxy functionality.

In certain embodiments, the polythioether of the present invention is present in the composition of the present invention in an amount of at least 30 weight percent, such as least 40 weight percent, or, in some cases, at least 45 weight percent, based on the total weight of non-volatile components in the composition. In certain embodiments, the polythioether of the present invention is present in the composition of the present invention in an amount of no more than 90 weight percent, such as no more than 80 weight percent, or, in some cases, no more than 75 weight percent, based on the weight of all non-volatile components of the composition.

As indicated, certain embodiments of the curable compositions of the present invention also comprise a curing agent. Curing agents useful in certain compositions of the invention (particularly in the case in which an uncapped thioether of the present invention is used) include epoxy resins, for example, hydantoin diepoxide, diglycidyl ether of bisphenol-A, diglycidyl ether of bisphenol-F, Novolactype epoxides, and any of the epoxidized unsaturated and phenolic resins, as well as unsaturated compounds, such as acrylic and methacrylic esters of commercially available polyols, unsaturated synthetic or naturally occurring resin compounds, triallylcyanurate, and olefinic terminated derivatives of the polythioethers of the present invention.

Isocyanate functional compounds can also be useful curing agents in the compositions of the present invention (particularly in the case in which a capped thioether of the present invention comprising an amine and/or hydroxyl terminal group is used). Suitable isocyanate functional compounds include, but are not limited to, polymeric polyisocyanates, non-limiting examples of which include polyisocyanates having backbone linkages chosen from urethane linkages (-NH-C(O)-O-), thiourethane linkages (-NH-C(O)-S-), thiocarbamate linkages (-NH-C(S)-O-), dithiourethane linkages (-NH-C(S)-S-) and combinations thereof.

Amine functional compounds can also be useful curing agents in the compositions of the present invention (particularly in the case in which a capped thioether of the present invention comprising an isocyanate terminal group is used). Suitable amines include those described earlier in connection with the preparation of an amine functional thioether of the present invention.

In addition, in the case where an uncapped thioether of the present invention is used, useful cures can be obtained through oxidative coupling of the thiol groups using organic and inorganic peroxides (e.g., MnO₂) known to those skilled in the art. Selection of the particular curing agent may affect the T_{g} of the cured composition.

Depending on the nature of the thioether(s) used in the composition, the composition will often contain 90% to 150% of the stoichiometric amount, such as 95 to 125%, of the selected curing agent(s).

Fillers useful in the certain embodiments of the compositions of the present invention include those commonly used in the art, including conventional inorganic fillers, such as carbon black and calcium carbonate (CaCO₃), as well as lightweight fillers. Suitable lightweight fillers include, for example, those described in United States Patent No. 6,525,168 at col. 4, lines 23-55. In certain embodiments, the compositions include 5 to 60 weight percent of the filler or combination of fillers, such as 10 to 50 weight percent, based on the total weight of the composition.

As will be appreciated, the thioethers, curing agents and fillers employed in certain compositions of the invention, as well as optional additives as described below, should be selected so as to be compatible with each other. Selection of compatible ingredients for the inventive compositions can readily be performed by those skilled in the art without recourse to undue experimentation.

In certain embodiments, the compositions of the present invention are curable at a maximum temperature of 0°C (i.e., at a temperature of 0°C or lower), such as -10°C, or, in some cases, -20°C, and have a T_{g} when cured not higher than -55°C, such as not higher than -60°C, or, in some cases, not higher than -65°C.

In addition to the foregoing ingredients, certain compositions of the invention can optionally include one or more of the following: colorants, thixotropes, accelerators, retardants, adhesion promoters, solvents and masking agents, among other components.

As used herein, the term "colorant" means any substance that imparts color and/or other opacity and/or other visual effect to the composition. The colorant can be added to the coating in any suitable form, such as discrete particles, dispersions, solutions and/or flakes. A single colorant or a mixture of two or more colorants can be used in the coatings of the present invention.

Example colorants include pigments, dyes and tints, such as those used in the paint industry and/or listed in the Dry Color Manufacturers Association (DCMA), as well as special effect compositions. A colorant may include, for example, a finely divided solid powder that is insoluble but wettable under the conditions of use. A colorant can be organic or inorganic and can be agglomerated or non-agglomerated. Colorants can be incorporated into the coatings by use of a grind vehicle, such as an acrylic grind vehicle, the use of which will be familiar to one skilled in the art.

Example pigments and/or pigment compositions include, but are not limited to, carbazole dioxazine crude pigment, azo, monoazo, diazo, naphthol AS, salt type (flakes), benzimidazolone,, isoindolinone, isoindoline and polycyclic phthalocyanine, quinacridone, perylene, perinone, diketopyrrolo pyrrole, thioindigo, anthraquinone, indanthrone, anthrapyrimidine, flavanthrone, pyranthrone, anthanthrone, dioxazine, triarylcarbonium, quinophthalone pigments, diketo pyrrolo pyrrole red ("DPPBO red"), titanium dioxide, carbon black and mixtures thereof. The terms "pigment" and "colored filler" can be used interchangeably.

Example dyes include, but are not limited to, those that are solvent and/or aqueous based such as phthalo green or blue, iron oxide, bismuth vanadate, anthraquinone, perylene and quinacridone.

Example tints include, but are not limited to, pigments dispersed in water-based or water miscible carriers such as AQUA-CHEM 896 commercially available from Degussa, Inc., CHARISMA COLORANTS and MAXITONER INDUSTRIAL COLORANTS commercially available from Accurate Dispersions division of Eastman Chemical, Inc.

As noted above, the colorant can be in the form of a dispersion including, but not limited to, a nanoparticle dispersion. Nanoparticle dispersions can include one or more highly dispersed nanoparticle colorants and/or colorant particles that produce a desired visible color and/or opacity and/or visual effect. Nanoparticle dispersions can include colorants such as pigments or dyes having a particle size of less than 150 nm, such as less than 70 nm, or less than 30 nm. Nanoparticles can be produced by milling stock organic or inorganic pigments with grinding media having a particle size of less than 0.5 mm. Example nanoparticle dispersions and methods for making them are identified in U.S. Patent No. 6,875,800 B2. Nanoparticle dispersions can also be produced by crystallization, precipitation, gas phase condensation, and chemical attrition (i.e., partial dissolution). In order to minimize re-agglomeration of nanoparticles within the coating, a dispersion of resin-coated nanoparticles can be used. As used herein, a "dispersion of resin-coated nanoparticles" refers to a continuous phase in which is dispersed discreet "composite microparticles" that comprise a nanoparticle and a resin coating on the nanoparticle. Example dispersions of resin-coated nanoparticles and methods for making them are identified in United States Patent Application Publication 2005-0287348 A1, filed June 24, 2004, U.S. Provisional Application No. 60/482,167 filed June 24, 2003, and United States Patent Application Serial No. 11/337,062, filed January 20, 2006.

Example special effect compositions that may be used in the compositions of the present invention include pigments and/or compositions that produce one or more appearance effects such as reflectance, pearlescence, metallic sheen, phosphorescence, fluorescence, photochromism, photosensitivity, thermochromism, goniochromism and/or color-change. Additional special effect compositions can provide other perceptible properties, such as opacity or texture. In a non-limiting embodiment, special effect compositions can produce a color shift, such that the color of the coating changes when the coating is viewed at different angles. Example color effect compositions are identified in U.S. Patent No. 6,894,086. Additional color effect compositions can include transparent coated mica and/or synthetic mica, coated silica, coated alumina, a transparent liquid crystal pigment, a liquid crystal coating, and/or any composition wherein interference results from a refractive index differential within the material and not because of the refractive index differential between the surface of the material and the air.

In general, the colorant can be present in any amount sufficient to impart the desired visual and/or color effect. The colorant may comprise from 1 to 65 weight percent of the present compositions, such as from 3 to 40 weight percent or 5 to 35 weight percent, with weight percent based on the total weight of the compositions.

Thixotropes, for example silica, are often used in an amount from 0.1 to 5 weight percent, based on the total weight of the composition.

Cure catalysts known to the art, such as amines, often are present in an amount from 0.1 to 5 weight percent, based on the total weight of the composition. Specific examples of useful catalysts are, without limitation, 1,4-diazabicyclo[2.2.2]octane (DABCO®, commercially available from Air Products, Chemical Additives Division, Allentown, Pa.) and DMP-30® (an accelerant composition including 2,4,6-tris(dimethylaminomethyl)phenol, commercially available from Rohm and Haas. Philadelphia, Pa.). It has been surprisingly discovered, however, that certain embodiments of the present invention will cure at ambient conditions even in the absence of any such cure catalyst.

Retardants, such as stearic acid, likewise often are used in an amount from 0.1 to 5 weight percent, based on the total weight of the composition. Adhesion promoters, if employed, are often present in amount from 0.1 to 15 weight percent, based on the total weight of the composition. Suitable adhesion promoters include phenolics, such as METHYLON phenolic resin available from Occidental Chemicals, and organosilanes, such as epoxy, mercapto or amino functional silanes, such as Silquest A-187 and Silquest A-1100 available from Momentive Performance Materials. Masking agents, such as pine fragrance or other scents, which are useful in covering any low level odor of the composition, are often present in an amount from 0.1 to 1 weight percent, based on the total weight of the composition.

In certain embodiments, the compositions of the present invention comprise a plasticizer which, in at least some cases, may allow the composition to include polythioether(s) which have a higher T_{g} than would ordinarily be useful in an aerospace sealant. That is, use of a plasticizer may effectively reduce the T_{g} of the composition, and thus increase the low-temperature flexibility of the cured polymerizable composition beyond that which would be expected on the basis of the T_{g} of the thioethers alone. Plasticizers that are useful in certain embodiments of the compositions of the present invention include, for example, phthalate esters, chlorinated paraffins, and hydrogenated terphenyls. The plasticizer or combination of plasticizers often constitute 1 to 40 weight percent, such as 1 to 10 weight percent of the composition. In certain embodiments, depending on the nature and amount of the plasticizer(s) used in the composition, thioethers of the invention which have T_{g} values up to -50°C, such as up to -55°C, can be used.

In certain embodiments, the compositions of the present invention can further comprise one or more organic solvents, such as isopropyl alcohol, in an amount ranging from, for example, 0 to 15 percent by weight on a basis of total weight of the composition, such as less than 15 weight percent and, in some cases, less than 10 weight percent.

In certain embodiments, however, the compositions of the present invention are substantially free or, in some cases, completely free, of any solvent, such as an organic solvent or an aqueous solvent, i.e., water. Stated differently, in certain embodiments, the compositions of the present invention are substantially 100 % solids.

In certain embodiments, the compositions, such as the previously described sealant compositions, are embodied as multi-pack compositions, such as two-pack compositions, wherein one package comprises the previously described polythioether and the second pack comprises the curing agent. The previously described additives and other materials can be added to either package as desired or necessary. The two packages are simply mixed together at or near the time of use.

The compositions of the present invention can be applied to any of a variety of substrates. Common substrates to which the compositions of the present invention are applied can include titanium, stainless steel, aluminum, anodized, primed, organic coated and chromate coated forms thereof, epoxy, urethane, graphite, fiberglass composite, KEVLAR®, acrylics and polycarbonates.

The compositions of the present invention can be applied directly onto the surface of a substrate or over an underlayer by any suitable coating process known to those of ordinary skill in the art.

In certain embodiments, the compositions of the present invention are fuel-resistant. As used herein, the term "fuel resistant" means that the compositions of the present invention, when applied to a substrate and cured, can provide a cured product, such as a sealant, that has a percent volume swell of not greater than 40%, in some cases not greater than 25%, in some cases not greater than 20%, in yet other cases not more than 10%, after immersion for one week at 140°F (60°C) and ambient pressure in jet reference fluid (JRF) Type I according to methods similar to those described in ASTM D792 or AMS 3269. Jet reference fluid JRF Type I, as employed herein for determination of fuel resistance, has the following composition (see AMS 2629, issued Jul. 1, 1989), §3.1.1 et seq., available from SAE (Society of Automotive Engineers, Warrendale, Pa.):

| | |
|---|---|
| Toluene | 28 ± 1% by volume |
| Cyclohexane (technical) | 34 ± 1% by volume |
| Isooctane | 38 ± 1% by volume |
| Tertiary dibutyl disulfide (doctor sweet) | 1 ± 0.005% by volume |

In certain embodiments, sealant compositions of the present invention provide a cured product, such as a sealant, having an elongation of at least 100% and a tensile strength of at least 400 psi when measured in accordance with the procedure described in AMS 3279, § 3.3.17.1, test procedure AS5127/1, § 7.7. Indeed, it was a surprising discovery that the polyfunctional polythioethers of the present invention can have substantially improved sealant properties, such as elongation, and/or tensile strength, as compared to prior art polyfunctional polythioethers.

In certain embodiments, sealant compositions of the present invention provide a cured product, such as a sealant having a lap shear strength of greater than 200 psi, in some cases at least 400 psi when measured according to the procedure described in SAE AS5127/1 paragraph 7.8.

As should be apparent from the foregoing description, the present invention is also directed to methods for sealing an aperture utilizing a composition of the present invention. These methods comprise (a) applying a composition of the present invention to a surface to seal the aperture; and (b) allowing the composition to cure under, for example, ambient conditions. As will also be appreciated, the present invention is also directed to aerospace vehicles comprising at least one surface coated with a coating composition of the present invention as well as aerospace vehicles comprising at least one aperture that is sealed with a sealant composition of the present invention.

Illustrating the invention are the following examples, which, however, are not to be considered as limiting the invention to their details. Unless otherwise indicated, all parts and percentages in the following examples, as well as throughout the specification, are by weight.

### EXAMPLES

### Comparative Example 1

12.6 grams of triallylcyanurate ("TAC") was placed into a first flask with 454.17 grams of 1,8-dimercapto-3,6-dioxaoctane ("DMDO"). 0.007 grams of a 50% KOH solution was placed into the flask. The mixture was then heated to 155-165°F and agitated. In a second flask, 0.40 grams of Vazo-67 (2,2'-azobis(2-methylbutyronitrile)) (available from E. I. du Pont de Nemours and Company) was added to 340.77 grams of diethyleneglycol divinylether ("DEG-DVE"). The Vazo-67 and DEG-DVE were mixed to form a DEG-DVE mixture. The DEG-DVE mixture was added to the first flask over six hours. The addition began after the temperature in the first flask reached 160°F, and continued while a temperature of 160-163°F was maintained. After the DEG-DVE mixture was completely added to the first flask, 0.56 grams of Vazo-67 was added in intervals of 0.14 grams per hour until the desired mercaptan equivalent weight was reached. Then, the temperature in the first flask was raised to 200-210°F for two hours. The first flask was then cooled to 170°F and placed under vacuum for one hour. The resulting compound had a mercaptan equivalent weight of 1696, a mercaptan functionality of 2.21, and a viscosity of 80 poise.

### Comparative Example 2

36.12 grams (0.05 mole) CAPCURE® 3-800 (a trithiol represented by Formula VIII in which R is an aliphatic hydrocarbon and "n" has a value of 1-2) and 424.58 grams of DMDO (2.33 moles) were charged in a 1-liter 4-neck round-bottom flask.

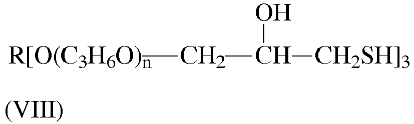

The flask was equipped with a stirrer, gas-passing adapter and thermometer. Stirring was started and the flask was flushed with dry nitrogen and the reaction mixture was heated to 169°F. A solution of radical initiator Vazo-67 (0.4 grams) in diethylene glycol divinyl ether (349.0 grams, 2.21 moles) was introduced in the reaction mixture over a period of 4 hours during which a temperature of 158-169°F was maintained. Eleven portions of Vazo-67 (∼0.151 grams each) were added at 1 hour intervals while the temperature was maintained at 158-169°C. The reaction mixture was heated at 212°F for 2 hours, cooled to 176°F and evacuated at 172-176°F/4-5mmHg for 1 hour. The resulting polymer (theoretical functionality: 2.21) had a mercaptan equivalent weight of 1536, and a viscosity of 171 poise.

### Example 1

15.21 grams of 1,3,5-tris(2-mercaptoethyl)-[1,3,5]-triazine-2,4,6-trione ("METT") (represented by Formula IX) was placed into a first flask with 435.78 grams of DMDO. 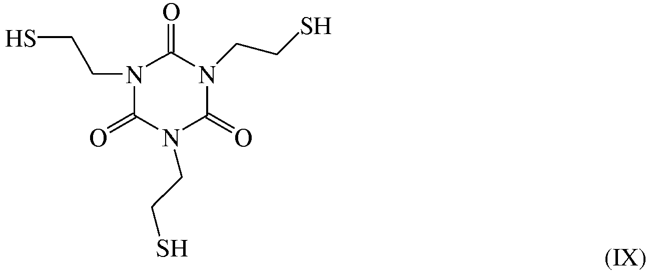 0.007 grams of a 50% KOH solution was placed into the flask. The mixture was then heated to 155-165°F and agitated. In a second flask, 0.43 grams of Vazo-67 was added to 349.70 grams of DEG-DVE. The Vazo-67 and DEG-DVE were mixed to form a DEG-DVE mixture. The DEG-DVE mixture was added to the first flask over six hours while a temperature of 155-165°F was maintained. After the DEG-DVE mixture was completely added to the first flask, 0.075 grams of Vazo-67 was added at a temperature of about 165°F. Then, about 1.4 grams of Vazo-67 was added at intervals of about 0.14 grams per hour at a temperature of between 168-172°F until the desired mercaptan equivalent weight was reached. Then, the temperature in the first flask was raised to 200-210°F for two hours. The first flask was then cooled to 170°F and placed under vacuum for one hour. The resulting composition had a mercaptan equivalent weight of 1668, a mercaptan functionality of 2.21, and a viscosity of 72 poise.

### Preparation of Sealant Compositions

Sealant Compositions were prepared using the polythiothers of Comparative Example 1, Comparative Example 2, and Example 1 using the components and amounts of Table 1. Amounts are in weight percent, based on the total weight of the base and accelerator, respectively.

**TABLE 1**

| Ingredient | Example 2 | Comparative Example 3 | Comparative Example 4 |
|---|---|---|---|
| Base | | | |
| Tung oil | 0.77 | 0.77 | 0.77 |
| Triethylene diamine diazabicyclo (2,2,2) octane¹ | 1.05 | 1.05 | 1.05 |
| Example 1 | 59.44 | -- | -- |
| Comparative Example 1 | -- | 59.44 | -- |
| Comparative Example 2 | -- | -- | 59.44 |
| Phenolic resin adhesion promoter² | 0.82 | 0.82 | 0.82 |
| Phenolic/polysulfide adhesion promoter³ | 0.55 | 0.55 | 0.55 |
| TYZOR TBT | 0.28 | 0.28 | 0.28 |
| A-1100 silane⁵ | 0.11 | 0.11 | 0.11 |
| Titanium dioxide | 0.55 | 0.55 | 0.55 |
| Amorphous silica | 0.83 | 0.83 | 0.83 |
| Aluminum hydroxide | 5.55 | 5.55 | 5.55 |
| Precipitated calcium carbonate | 30.05 | 30.05 | 30.05 |

| Accelerator | | | |
|---|---|---|---|
| EPON 828⁶ | 26.53 | 26.53 | 26.53 |
| DEN 431⁷ | 17.68 | 17.68 | 17.68 |
| HB-40⁸ | 10.70 | 10.70 | 10.70 |
| Calcium carbonate | 42.44 | 42.44 | 42.44 |
| Carbon black | 0.22 | 0.22 | 0.22 |
| Carbamate salt⁹ | 0.088 | 0.088 | 0.088 |
| Epoxy silane¹⁰ | 2.25 | 2.25 | 2.25 |
| Deionized water | 0.085 | 0.085 | 0.085 |
| Diphenylguanidine | 0.011 | 0.011 | 0.011 |

| | | | |
|---|---|---|---|
| ¹ Available from Air Products & Chemicals. ² METHYLON 75108 available from Occidental Chemical. ³ The phenolic/polysulfide adhesion promoter was prepared by reacting about 31% VARCUM 29202 phenolic resin, 66% Thiokol LP-3 polysulfide and 3% of a polymer prepared according to Example 4 of U.S. Pat. No. 4,623,711 (at a ratio of 1 mole dithiol to 1 mole polysulfide) at a temperature of about 150°F (65°C) for 45 minutes, then heating to 230°F (110° C) over a 45-60 minute period, then heating at 230°F (110°C) for 165 minutes. ⁴ Titanate available from E.I. duPont de Nemours Company. ⁵ Amino functional silane available from Momentive Performance Materials. ⁶ Bisphenol A diglycidyl ether available from Hexicon. ⁷ Epoxy novolac available from Dow Chemical. ⁸ Plasticizer available from Solvay. ⁹ Ferbam 76% WDG carbamate salt available from Cabot Corp. ¹⁰ A-187 epoxy functional silane available from Momentive Performance Materials. | | | |

The compounded base composition was mixed intimately with the accelerator composition in the equivalent weight ratio of 1.0:1.1 and cured at ambient temperature and humidity for 2 days, followed by 1 day at 140°F (hereinafter referred to as "dry"). The compositions were then tested to determine tensile strength, elongation, and peel strength. Results are set forth in Table 2.

**TABLE 2**

| Sealant Composition | Tensile Strength (dry)¹ | % Elongation (dry)¹ | Peel Strength² |
|---|---|---|---|
| Comparative Example 3 | 355 psi | 275% | Alodine Substrate |
| | | | 57(100)³ |
| | | | 35(100)⁴ |
| | | | 44(100)/49(100)⁵ |
| | | | MIL C-27725 Substrate |
| | | | 52(100)³ |
| | | | 32(100)⁴ |
| Comparative Example 4 | 452 psi | 121% | Alodine Substrate |
| | | | 4-8(100)³ |
| | | | 4-8(100)⁴ |
| | | | 4-8(100)/4-8(100)⁵ |
| | | | MIL C-27725 Substrate |
| | | | 4-8(100)³ |
| | | | 4-8(100)⁴ |
| Example 3 | 447 psi⁶ | 564%⁶ | Alodine Substrate |
| | | | 53(100)³ |
| | | | 50(100)⁴ |
| | | | 28(100)/18(20)⁵ |
| | | | MIL C-27725 Substrate |
| | | | 54(100)³ |
| | | | 50(100)⁴ |
| | | | 46(100)/46(100)⁵ |

| | | | |
|---|---|---|---|
| ¹ Tested according to SAE AS5127/1B Paragraph 7.7. ² Tested according to AMS 3265B Paragraph 8.1. Results reported as pounds per linear inch(pli)/(% cohesion) ³ Dry sample ⁴ Tested following immersion of sample in Jet Reference Fuel Type I for 7 days at 140°F according to SAE AS5127/1B Paragraph 8.1. ⁵ Tested following immersion of sample in Jet Reference Fuel Type I/Solution of 3% NaCl for 7 days at 140°F according to SAE 5127/1B Paragraph 8.1. ⁶ Reported result was an average of three samples. | | | |

As shown by these results, a sealant composition comprising a polythioether polymer according to an embodiment of the present invention has increased tensile strength and elongation. Furthermore, compositions of the present invention do not have compromised peel strength.

Whereas particular embodiments of this invention have been described above for purposes of illustration, it will be evident to those skilled in the art that numerous variations of the details of the present invention may be made without departing from the invention as defined in the appended claims.

## Claims

1. A polythioether comprising the reaction product of reactants comprising:
a) an isocyanurate-containing trithiol;
b) a polythiol different from (a); and
c) a diene,
wherein the polythiol (b) comprises a dithiol comprising a compound represented by formula:
HS-R-SH
where R is -[(-CH₂-)ₚ-O-]_{q}-(-CH₂-)ᵣ- or -[(-CH₂-)ₚ-S-]_{q}-(-CH₂-)ᵣ-, p is an integer having a value ranging from 2 to 6, q is an integer having a value ranging from 1 to 5, and r is an integer having a value ranging from 2 to 10.

2. The polythioether of claim 1, wherein the isocyanurate-containing trithiol has the structure: 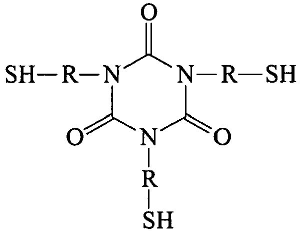 wherein each R represents a divalent linking group and may be the same or different.

3. The polythioether of claim 2, wherein
i) each R represents a straight or branched chain alkylene, preferably ethylene; or
ii) each R comprises a urethane linkage.

4. The polythioether of claim 1, wherein
i) the isocyanurate-containing trithiol is substantially free from ester linkages; or
ii) the polythioether has an average functionality of from 2.1 to 2.8.

5. The polythioether of claim 1, wherein the diene comprises a compound represented by the formula:
CH₂=CH-O-(-R⁵-O-)ₘ-CH=CH₂
where R⁵ is a C₂₋₆ n-alkylene group, a C₂₋₆ branched alkylene group, a C₆₋₈ cycloalkylene group, a C₆₋₁₀ alkylcycloalkylene group, or -[(-CH₂-)ₚ-O-]_{q}-(-CH₂-)ᵣ-, where p is an integer having a value ranging from 2 to 6, q is an integer having a value ranging from 1 to 5, and r is an integer having a value ranging from 2 to 10.

6. The polythioether of any of claims 1 or 5, wherein the diene is a divinylether.

7. A polythioether comprises a compound represented by the formula: 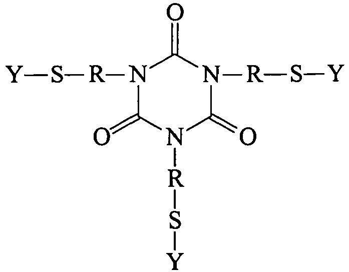 wherein: (A) each Y, which may be the same or different, is a repeat unit comprising a thioether linkage; and (B) each R represents a divalent linking group and may be the same or different, wherein each Y includes the structure:
-[-(CH₂)₂-O-(R²-O)ₘ-(CH₂)₂-S-R¹-S-]ₙ-
wherein:
R¹ is selected from -[(-CH₂)ₚ-X]_{q}(-CH₂)ᵣ- groups:
R² is selected from C₂ to C₁₀ n-alkylene groups, C₂ to C₆ branched alkylene groups, C₆ to C₈ cycloalkylene groups, C₆ to C₁₄ alkylcycloalkylene groups, heterocyclic groups, and -[(-CH₂)ₚ-X]_{q}-(-CH₂)ᵣ-;
X is selected from O atoms, and S atoms;
p is an integer from 2 to 6;
q is an integer from 1 to 5;
r is an integer from 2 to 10;
m has a value of from 0 to 10; and
n is an integer from 1 to 60.

8. The polythioether of claim 7, wherein each R represents a straight or branched chain alkylene.

9. An isocyanurate-containing trithiol having the formula: 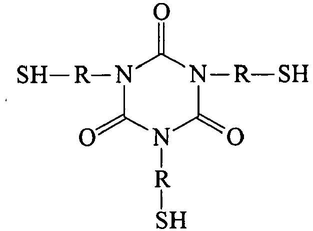 wherein each R, which may be the same or different, represents a divalent linking group comprising a urethane linkage.

10. A composition comprising the polythioether of claim 1 and at least one of a curing agent and a filler.

11. An aerospace vehicle comprising at least one aperture sealed with a sealant deposited from the composition of claim 104.

## Patentansprüche

1. Polythioether enthaltend das Reaktionsprodukt der Reaktanten, die:
a) ein isocyanurathaltiges Trithiol,
b) ein Polythiol, das sich von (a) unterscheidet, und
c) ein Dien
enthalten, wobei das Polythiol (b) ein Dithiol enthält, das eine Verbindung, die durch die Formel
HS-R-SH
wiedergegeben ist, enthält,
worin R gleich -[(-CH₂-)ₚ-O-]_{q}-(-CH₂-)ᵣ- oder -[(-CH₂-)ₚ-S-]_{q}-(-CH₂-)ᵣ- ist, p eine ganze Zahl mit einem Wert im Bereich von 2 bis 6 ist, q eine ganze Zahl mit einem Wert im Bereich von 1 bis 5 ist und r eine ganze Zahl mit einem Wert im Bereich von 2 bis 10 ist.

2. Polythioether nach Anspruch 1, wobei das isocyanurathaltige Trithiol die Struktur 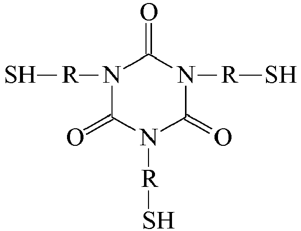 aufweist, worin jedes R für eine divalente Verbindungsgruppe steht und gleich oder unterschiedlich sein kann.

3. Polythioether nach Anspruch 2, worin
i) jedes R für ein geradkettiges oder verzweigtes Alkylen steht, vorzugsweise Ethylen, oder
ii) jedes R eine Urethanverknüpfung aufweist.

4. Polythioether nach Anspruch 1, wobei
i) das isocyanurathaltige Thrithiol im Wesentlichen frei von Esterverknüpfungen ist oder
ii) der Polythioether eine mittlere Funktionalität von 2,1 bis 2,8 aufweist.

5. Polythioether nach Anspruch 1, wobei die Dienverbindung eine Verbindung enthält, die durch die Formel
CH₂=CH-O-(-R⁵-O-)ₘ-CH=CH₂
wiedergegeben ist, worin
R⁵ eine C₂₋₆-n-Alkylengruppe, eine verzweigte C₂₋₆-Alkylengruppe, eine C₆₋₈-Cycloalkylengruppe, eine C₆₋₁₀-Alkylcycloalkylengruppe oder -[(-CH₂-)ₚ-O-]_{q}-(-CH₂-)ᵣ- ist, worin p eine ganze Zahl mit einem Wert im Bereich von 2 bis 6 ist, q eine ganze Zahl mit einem Wert von 1 bis 5 ist und r eine ganze Zahl mit einem Wert im Bereich von 2 bis 10 ist.

6. Polythioether nach einem der Ansprüche 1 oder 5, wobei das Dien ein Divinylether ist.

7. Polythioether enthaltend eine Verbindung, die durch die Formel 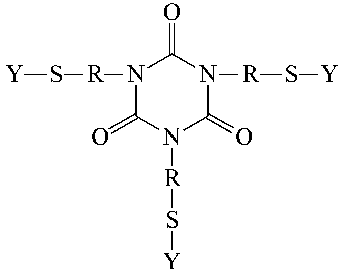 wiedergegeben ist, worin: (A) jedes Y gleich oder unterschiedlich sein kann, eine Wiederholungseinheit ist, die eine Thioetherverknüpfung enthält, und (B) jedes R für eine divalente Verbindungsgruppe steht und gleich oder unterschiedlich sein kann, wobei jedes Y die Struktur
-[-(CH₂)₂-O-(R²-O)ₘ-(CH₂)₂-S-R¹-S-]ₙ-,
enthält, worin
R¹ ausgewählt ist aus -[(-CH₂)ₚ-X]_{q}-(-CH₂)ᵣ- -Gruppen,
R² ausgewählt ist aus C₂-C₁₀-n-Alkylengruppen, verzweigten C₂-C₆-Alkylengruppen, C₆-C₈-Cycloalkylengruppen, C₆-C₁₄-Alkylcycloalkylengruppen, heterocyclischen Gruppen und -[(-CH₂)ₚ-X]_{q}-(-CH₂)ᵣ-,
X ausgewählt ist aus O-Atomen und S-Atomen,
p eine ganze Zahl von 2 bis 6 ist,
q eine ganze Zahl von 1 bis 5 ist,
r eine ganze Zahl von 2 bis 10 ist,
m einen Wert von 0 bis 10 aufweist und
n eine ganze Zahl von 1 bis 60 ist.

8. Polythioether nach Anspruch 7, wobei jedes R für ein gerad- oder verzweigtkettiges Alkylen steht.

9. Isocyanurathaltiges Trithiol mit der Formel 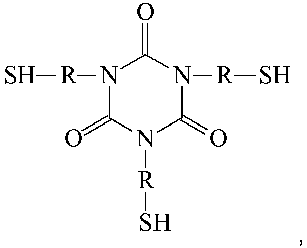 worin jedes R, das gleich oder unterschiedlich sein kann, für eine divalente Verbindungsgruppe steht, die eine Urethanverknüpfung aufweist.

10. Zusammensetzung enthaltend den Polythioether nach Anspruch 1 und wenigstens eins von einem Härtungsmittel und einem Füllstoff.

11. Luft- oder Raumfahrzeug enthaltend wenigstens eine Öffnung, die mit einem Dichtungsmittel, abgeschieden aus der Zusammensetzung nach Anspruch 10, abgedichtet ist.

## Revendications

1. Polythioéther comprenant le produit de réaction de réactifs qui comprennent :
a) un isocyanurate-trithiol,
b) un polythiol différent du composant (a),
c) et un diène,
pour lequel le polythiol (b) comprend un dithiol comprenant un composé représenté par la formule
HS-R-SH
dans laquelle R représente un groupe de formule -[(-CH₂-)ₚ-O-]_{q}-(-CH₂-)ᵣ- ou -[(-CH₂-)ₚ-S-]_{q}-(-CH₂-)ᵣ où l'indice p est un nombre entier valant de 2 à 6, l'indice q est un nombre entier valant de 1 à 5, et l'indice r est un nombre entier valant de 2 à 10.

2. Polythioéther conforme à la revendication 1, dans lequel l'isocyanurate-trithiol présente la structure suivante : 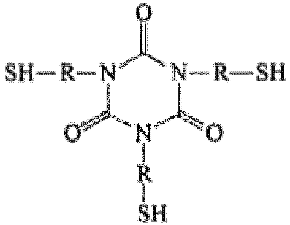 dans laquelle chaque symbole R représente un groupe de raccordement divalent, et ces groupes peuvent être identiques ou différents.

3. Polythioéther conforme à la revendication 2, dans lequel
i) chaque symbole R représente un groupe alcanediyle à chaîne linéaire ou ramifiée, de préférence un groupe éthanediyle,
ii) ou chaque symbole R représente un groupe comprenant un raccord de type uréthane.

4. Polythioéther conforme à la revendication 1,
i) dans lequel l'isocyanurate-trithiol ne comporte pratiquement pas de raccords de type ester,
ii) ou lequel polythioéther présente un nombre moyen de groupes fonctionnels valant de 2,1 à 2,8.

5. Polythioéther conforme à la revendication 1, dans lequel le diène comprend un composé représenté par la formule
CH₂=CH-O-(-R⁵-O-)ₘ-CH=CH₂
dans laquelle R⁵ représente un groupe n-alcanediyle en C₂₋₆, un groupe alcanediyle ramifié en C₂₋₆, un groupe cycloalcanediyle en C₆₋₈, un groupe alkyl-cycloalcanediyle en C₆₋₁₀, ou un groupe de formule
-[(-CH₂-)ₚ-O-]_{q}-(-CH₂-)ᵣ-
où l'indice p est un nombre entier valant de 2 à 6, l'indice q est un nombre entier valant de 1 à 5, et l'indice r est un nombre entier valant de 2 à 10.

6. Polythioéther conforme à l'une des revendications 1 et 5, dans lequel le diène est un bis(vinyl-éther).

7. Polythioéther comprenant un composé représenté par la formule : 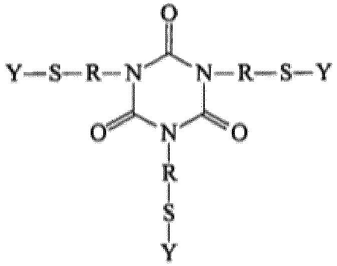 dans laquelle
A) chaque symbole Y représente un reste à motifs répétés comprenant un raccord de type thioéther, et ces restes peuvent être identiques ou différents,
B) et chaque symbole R représente un groupe de raccordement divalent, et ces groupes peuvent être identiques ou différents,
étant entendu que chaque symbole Y représente un groupe comportant la structure suivante :
-[-(CH₂)₂-O-(R²-O)ₘ-(CH₂)₂-S-R¹-S-]ₙ-
dans laquelle
- R¹ représente un groupe choisi parmi ceux de formule -[(-CH₂-)ₚ-X-]_{q}-(-CH₂-)ᵣ-,
- R² représente un groupe choisi parmi les groupes n-alcanediyle en C₂-C₁₀, alcanediyle ramifié en C₂-C₆, cycloalcanediyle en C₆-C₈, et alkyl-cycloalcanediyle en C₆-C₁₄, les groupes hétérocycliques et les groupes de formule -[(-CH₂-)ₚ-X-]_{q}-(-CH₂-)ᵣ-,
- X représente un atome d'oxygène ou de soufre,
- l'indice p est un nombre entier valant de 2 à 6,
- l'indice q est un nombre entier valant de 1 à 5,
- l'indice r est un nombre entier valant de 2 à 10,
- l'indice m vaut de 0 à 10,
- et l'indice n est un nombre entier valant de 1 à 60.

8. Polythioéther conforme à la revendication 7, dans lequel chaque symbole R représente un groupe alcanediyle à chaîne linéaire ou ramifiée.

9. Isocyanurate-trithiol de formule : 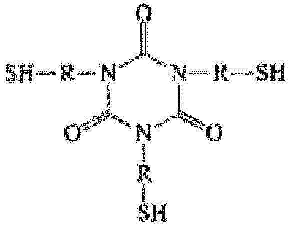 dans laquelle chaque symbole R représente un groupe de raccordement divalent comportant un raccord de type uréthane, et ces groupes peuvent être identiques ou différents.

10. Composition comprenant un polythioéther conforme à la revendication 1, et au moins l'un des composants suivants : un agent durcisseur et une charge.

11. Véhicule aérospatial comprenant au moins une ouverture scellée avec un joint d'étanchéité formé à partir d'une composition conforme à la revendication 10.
